# EUROPEAN PATENT APPLICATION

(11) **EP 2 497 824 A1**
(43) Date of publication of application: **12.09.2012**
(21) Application number: 10826846.7
(22) Date of filing: 29.10.2010
(51) Int. Cl.: C12N 5/071, C12M 1/40, C12M 3/00, G01N 37/00

(54) **METHOD FOR PREPARING SUBSTRATE FOR ARRAYING ANIMAL CELLS THEREON AND METHOD FOR PREPARING SUBSTRATE HAVING ANIMAL CELLS ARRAYED THEREON**

(30) Priority: 30.10.2009 JP 2009250516
(71) Applicant: Japan Agency for Marine-Earth Science and Technology, Yokosuka-shi Kanagawa 237-0061 (JP)
(72) Inventor: KOYAMA, Sumihiro, Yokosuka-shi Kanagawa 237-0061 (JP)
(74) Representative: Vossius & Partner
(86) International application number: PCT/JP2010/069271
(87) International publication number: WO 2011/052716

(57) **Abstract**

A new means of separately arranging individual animal cells on a substrate surface is provided. A method for preparing a substrate for arranging animal cells in an array, comprising steps (1) to (3): (1) preparing a substrate having adsorption surfaces in an array on an electrode substrate surface; (2) causing an extracellular matrix to adsorb to the electrode surface and the adsorption surfaces of the substrate; and (3) applying a weak potential to the electrode to cause the extracellular matrix on the electrode surface to separate to obtain a substrate with the extracellular matrix adhered to the adsorption surfaces thereof. A method for preparing a substrate on which animal cells have been arranged in an array, comprising steps (1) to (4): (1) preparing a substrate having adsorption surfaces in an array on an electrode substrate surface; (2) causing an extracellular matrix to adsorb to the electrode surface and the adsorption surfaces of the substrate; (3) applying a weak potential to the electrode to cause the extracellular matrix on the electrode surface to separate to obtain a substrate with extracellular matrix adhered to the adsorption surfaces thereof; and (4) culturing the animal cells on the surface of the substrate obtained in (3) to obtain a substrate on which the animal cells have adhered to the adsorption surface.

## Description

### [Cross-Reference to Related Patent Applications]

The present application claims priority under Japanese Patent Application 2009-250516, filed on October 30, 2009, the contents of the entirety of which are incorporated herein by reference.

### [Technical Field]

The present invention relates to a method for preparing a substrate for arranging animal cells in an array, and to a method for preparing a substrate on which animal cells are arranged in an array.

### [Background Art]

Attempts have been made to specify, select, and use cells that have been selected at the single-cell level. For example, the detection of the antigen specificity of an individual lymphocyte, the recovery of an individual antigen specific lymphocyte that has been detected, and the use of an individual antigen specific lymphocyte that has been recovered to, for example, produce antibodies are being examined (Patent Reference 1).

Patent Reference 1 employs a microwell array chip to detect antigen specific lymphocytes, The microwell array chip has dimensions and is of a shape such that each microwell holds just one lymphocyte.

It is known that when animal cells are cultured on a substrate surface, an adhesive protein known as extracellular matrix forms outside the cells, adhering the cells to the substrate surface. The separation for use of cells that have adhered to a substrate surface in this manner is also known. The use of electric stimulation is known as a separation method (Patent References 2 to 4).

### [Prior Art References]

### [Patent References]

[Patent Reference 1] Japanese Unexamined Patent Publication (KOKAI) No. 2004-173681
[Patent Reference 2] Japanese Unexamined Patent Publication (KOKAI) No. 2008-295382
[Patent Reference 1] Japanese Unexamined Patent Publication (KOKAI) No. 2005-312343
[Patent Reference 1] Japanese Unexamined Patent Publication (KOKAI) Heisei No. 10-42857

The entire contents of Patent References 1 to 4 are hereby incorporated by reference.

### [Summary of the Invention]

### [Problems to Be Solved by the Invention]

However, the adhesion of cells to the substrate surface by means of the extracellular matrix and the separation of cells by electric stimulus are not employed as means of arranging individual animal cells on a substrate surface.

The object of the present invention is to provide a new means permitting the arrangement of individual animal cells at the single-cell level on a substrate surface.

### [Means of Solving the Problem]

The present inventors conducted extensive research into achieving the above object, resulting in the discovery that by means of extracellular matrix adsorption and partial electric stimulus of the extracellular matrix, it was possible to form a culture substrate to which individual animal cells could be made to adhere. The present invention was devised on that basis.

The present invention is as set forth below:
[1] A method for preparing a substrate for arranging animal cells in an array, comprising the steps of:
   (1) preparing a substrate having arrayed adsorption surfaces on an electrode substrate surface;
   (2) causing an extracellular matrix to adsorb to the electrode surface and the adsorption surfaces of the substrate; and
   (3) applying a weak potential to the electrode to cause the extracellular matrix on the electrode surface to separate to obtain a substrate with the extracellular matrix adhered to the adsorption surfaces thereof.
[2] A method for preparing a substrate on which animal cells have been arranged in an array, comprising the steps of:
   (1) preparing a substrate having adsorption surfaces in an array on an electrode substrate surface;
   (2) causing an extracellular matrix to adsorb to the electrode surface and the adsorption surfaces of the substrate;
   (3) applying a weak potential to the electrode to cause the extracellular matrix on the electrode surface to separate to obtain a substrate with the extracellular matrix adhered to the adsorption surfaces thereof;
   (4) culturing the animal cells on the surface of the substrate obtained in (3) to obtain a substrate on which the animal cells have adhered to the adsorption surfaces thereof.
[3] The preparation method according to [1] or [2], wherein the dimensions of each of the adsorption surfaces in the array permit the adhesion of a single animal cell to each adsorption surface.
[4] The preparation method according to any one of [1] to [3] wherein the substrate is selected from the group consisting of electrically non-conductive organic materials and inorganic materials.
[5] The preparation method according to any one of [1] to [3] wherein the substrate is glass, plastic, or ceramic.
[6] The preparation method according to any one of [1] to [4], wherein the weak potential in (3) falls within a range of -0.4 V to -0.2 V or +0.2 V to + 0.5 V (vs. Ag/Cl).
[7] The preparation method according to any one of [2] to [6], wherein the animal cell culturing in (4) is conducted while applying a potential falling within a range of -0.4 V to -0.2 V (vs. Ag/AgCl) to the electrode to inhibit adhesion of animal cells to the electrode.
[8] The preparation method according to any one of [2] to [6], wherein the animal cell culturing in (4) is conducted by applying a positive potential to the electrode to cause animal cells to adsorb to the electrode, yielding a substrate on which the animal cells have adhered to the adsorption surfaces and the electrode.

### [Effect of the Invention]

The present invention permits the formation of a culture substrate making it possible to cause individual animal cells to adhere. Using this culture substrate, it is possible to separately arrange individual animal cells on the substrate surface.

### [Brief Description of the Drawings]

[Figure 1] Descriptive drawings showing the electrode manufacturing sequence in Example 1.
[Figure 2] Descriptive drawings of steps (2) to (4).
[Figure 3] Images of the patterned electrode surface when employing FITC-labeled collagen I as the extracellular matrix protein.
[Figure 4] Images of the patterned electrode surface when employing FITC-labeled collagen I as the extracellular matrix protein.
[Figure 5] Images of the patterned electrode surface when employing FITC-labeled poly-L-lysine as the extracellular matrix protein.
[Figure 6] Images of the electrode surface following 24 hours of culturing of animal cells (HeLa cells), obtained in Example 2.
[Figure 7] Images of the electrode surface following 24 hours of culturing of animal cells (PC12 cells), obtained in Example 3.
[Figure 8] Results of measurement of the water contact angle of the surface of the ITO electrode in Reference Example 1.

### [Modes of Carrying Out the Invention]

The present invention relates to a method for preparing a substrate for arranging animal cells in an array, and to a method employing this substrate to prepare a substrate on which animal cells are arranged in an array. The method for preparing a substrate for arranging animal cells in an array comprises steps (1) to (3) below, and the method for preparing a substrate on which animal cells are arranged in an array comprises steps (1) to (4) below.

### Step (1)

Step (1) is a step of preparing a substrate having adsorption surfaces in an array on an electrode substrate surface. On the electrode substrate, the electrode is present over part or all of the surface of an insulating substrate. For example, it can be a glass slide that has been coated with indium [tin] oxide (ITO). However, the insulating substrate is not intended to be limited to glass slides, and the electrode is not intended to be limited to indium [tin] oxide (ITO).

On the electrode substrate, the adsorption surfaces are arranged in an array on the surface. The adsorption surfaces are surfaces for adsorbing extracellular matrix, other than the electrode, that are comprised of a material that is capable of the adsorbing an extracellular matrix. An example of a material that is capable of adsorbing an extracellular matrix is glass. However, there is no specific limitation other than that it be an electrically non-conductive solid. It can be an electrically non-conductive organic material or inorganic material. Examples of electrically non-conductive organic materials and inorganic materials other than glass are plastics and ceramics. Accordingly, a glass surface can be given as an example of an adsorption surface. However, limitation to a glass surface is not intended; a suitable selection can be made from among electrically non-conductive organic materials and inorganic materials. When observing the state of adhesion of cells and the adsorption of the extracellular matrix by microscopy or the like, the substrate is desirably transparent.

The term "in an array" means, for example, that the adsorption surfaces are arranged as multiple microregions in rows and columns. The number of microregions that are arranged in rows and columns is not specifically limited, and can be suitably determined based on the type (size) of the animal cells and the use objective of the substrate on which the animal cells are arranged in an array. For example, the number can fall within a range of 10 to 10⁵ vertically to 10 to 10⁵ horizontally. However, there is no intent to limit this range. The shape of the adsorption surfaces can be rectangular (triangular, rectangular, polyhedral, or the like), circular, elliptical, or the like, and can be suitably determined. The dimensions of the individual adsorption surfaces in an array permit the adhesion of a single animal cell to a single adsorption surface. The dimensions of the animal cells will vary with the cell; the dimensions of the adsorption surfaces can be suitably determined based on the dimensions of the animal cells that are being caused to adhere. However, when the animal cells are HeLa cells, the dimensions of the adsorption surfaces can fall within a range of 25 to 100 µm, for example. The dimensions of the individual adsorption surfaces in an array can also permit the adhesion of two or more animal cells to each adsorption surface.

The adsorption surfaces in an array can be formed on the surface of an electrode substrate, for example, by coating an electrode layer on a substrate surface, forming a mask for the adsorption surfaces in the form of an array on the surface of the electrode layer, etching the surface of the electrode layer through the mask, and removing the mask. Alternatively, the adsorption surfaces in an array can be formed on the surface of an electrode substrate by coating an electrode layer on the substrate surface through a mask for the adsorption surfaces in an array, and then removing the mask. The formation of the electrode layer and etching of the outer surface of the electrode layer can be suitably implemented by the usual methods.

### Step (2)

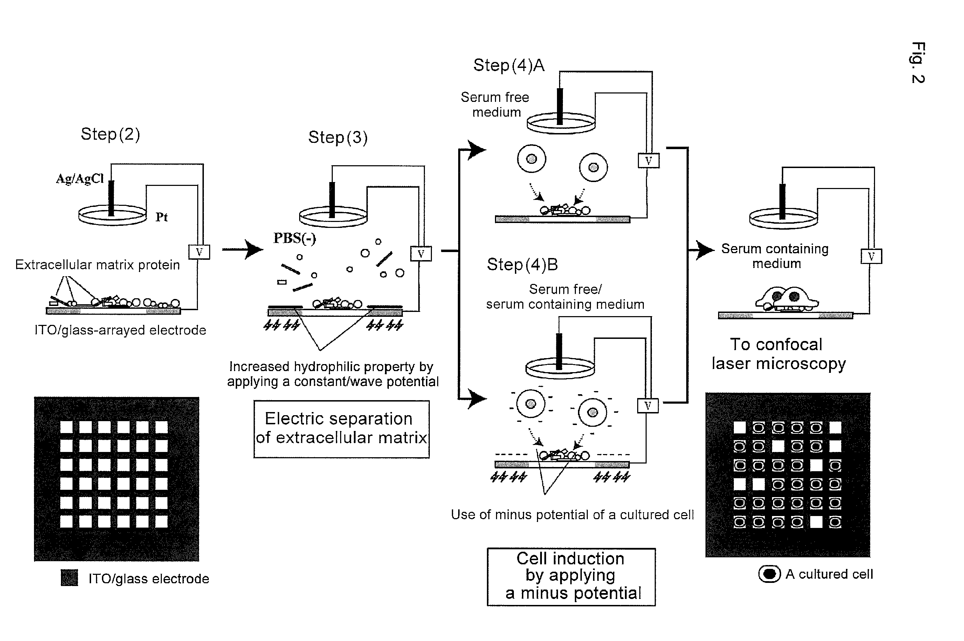

Step (2) is a step in which an extracellular matrix is caused to adsorb to the electrode surface and adsorption surfaces of the substrate prepared in step (1). As shown in step (2) in Figure 2, the extracellular matrix is caused to adsorb to the electrode surface and adsorption surfaces (array electrode surfaces) of the substrate.
The term "extracellular matrix" refers to a cellular adhesive protein such as collagen, proteoglycan, hyaluronic acid, fibronectin, laminin, tenascin, or entactin. The extracellular matrix is available in the form of commercial products. Adsorption of the extracellular matrix to the substrate surface can be achieved by coating an aqueous solution containing the extracellular matrix on the substrate surface, or by immersing the substrate surface in an aqueous solution containing an extracellular matrix while maintaining it facing upward, and allowing it to stand.

### Step (3)

Step (3) is a step in which a weak potential is applied to the electrode of the substrate onto which the extracellular matrix has been adsorbed to separate the extracellular matrix from the electrode surface and obtain a substrate on which the extracellular matrix has been adsorbed onto the adsorption surfaces thereof. As shown in step (3) in Figure 2, the extracellular matrix on the surface of the electrode to which the weak potential is applied is separated while maintaining adsorption of the extracellular matrix on the adsorption surfaces(central portion). By way of example, it is desirable for the weak potential to fall within a range of -0.4 V to -0.2 V or +0.2V to +0.5 V (vs. Ag/AgCl) from the perspectives of maintaining adsorption of the extracellular matrix that is adsorbed to the adsorption surfaces and permitting separation of the extracellular matrix from the electrode surface. By way of example, the weak potential that is applied within the range of -0.4 V to -0.2 V (vs. Ag/AgCl) is desirably suitably determined based on the quantity of extracellular matrix that has adsorbed onto the electrode surface and the type of extracellular matrix, and is applied for a period falling within a range of from 2 to 24 hours, for example. Following separation through the application of a weak potential, the substrate surface can be cleaned as needed and the free extracellular matrix can be removed.

Steps (1) to (3) permit the preparation of a substrate for arranging animal cells in an array. Here, the substrate for arranging animal cells in an array that is obtained can be further subjected to step (4) to manufacture a substrate on which animal cells are arranged in an array.

### Step (4)

Step (4) is a step in which the animal cells on the substrate surface obtained in step (3) are cultured to obtain a substrate on which the animal cells have adhered to adsorption surfaces. As shown in step (4)A of Figure 2, the animal cells have adhered to the adsorption surfaces. The animal cells that are cultured on the substrate surface are not specifically limited. Examples are normal human cells, ES cells, iPS cells, HeLa cells, and CHO cells. Known culturing conditions can be suitably adopted to culture the animal cells on the substrate surface based on the type of animal cell. When a culture solution containing animal cells is supplied to the substrate surface and culturing is conducted under conditions based on the type of animal cells, since the extracellular matrix is adsorbed onto the adsorption surfaces, the animal cells adhere to the extracellular matrix and thus adhere to the adsorption surfaces. Thus, a substrate is prepared on which animal cells are arranged in an array.

The culturing of animal cells in step (4) can be conducted by applying a potential falling within a range of -0.4 V to -0.2 V (vs. Ag/AgCl) to the electrode while inhibiting the adhesion of animal cells to the electrode. As shown in step (4)B in Figure 2, by applying a voltage falling within a range of -0.4 V to -0.2 V (vs. Ag/AgCl) to the electrode, adhesion of animal cells to the electrode is inhibited and animal cells adhere to the adsorption surfaces. For example, when culturing is conducted in serum-containing medium, since extracellular matrix protein is contained in serum-containing medium, the application of a negative potential of -0.2 V to -0.4 V (vs. Ag/AgCl) during culturing as well inhibits the adsorption of extracellular matrix protein to the electrode. As a result, adhesion of animal cells to the electrode is inhibited. Even when culturing is conducted without serum, the extracellular matrix that is secreted by animal cells will sometimes cause the animal cells to adhere to the electrode. Even in that case, applying a negative potential of -0.2 V to -0.4 V (vs. Ag/AgCl) even during culturing inhibits the adsorption of extracellular matrix protein to the electrode and inhibits adhesion of the animal cells to the electrode. Further, since animal cells have a negative charge, the adhesion of animal cells to an electrode substrate surface to which a negative potential is being applied can also be inhibited. Thus, it is possible to cause cells to adhere to just portions of the array pattern where the adhesion of animal cells is desirable without the adhesion of animal cells onto the surface of an electrode to which a negative potential is being applied.

In the culturing of animal cells in step (4), it is also possible to apply a positive potential to the electrode to cause the animal cells to adhere to the electrode and obtain a substrate on which the animal cells have adhered to both the electrode and the adsorption surfaces. Since animal cells have a negative charge as set forth above, the electrostatic attraction of the surface of an electrode substrate to which a positive potential is being applied causes the animal cells to adhere. Thus, the application of a positive charge to the electrode makes it possible to cause cells to adhere well to spots that have not been coated with an extracellular matrix protein such as collagen. From the perspectives of not damaging the animal cells and inducing the adhesion of animal cells by means of electrostatic attraction, the positive potential is, for example, suitably +0.2 V to + 1.0 V (vs. Ag/AgCl), desirably falling within a range of +0.2 V to +0.6 V (vs. Ag/AgCl),

The substrate on which animal cells are arranged in an array that is prepared by the method of the present invention can be used, for example, to examine the properties of animal cells and to examine the substances that are discharged by animal cells. In particular, by arranging the animal cells one by one in an array on the surface of a culture substrate, it is possible to employ physicochemical stimulation to analyze the mechanisms that are activated at the single-cell level. Further, the cells that adhere to the electrode substrate surface and the adsorption surfaces in the vicinity of the electrode substrate surface can be caused to separate by applying to the electrode a high-frequency wave potential (with, for example, a rectangular, sinusoidal, or triangular waveform) with a frequency falling within a range of 1 KHz to 10 MHz and a potential of ± 1.0 V (vs. Ag/AgCl) or lower. However, the use of a phosphate buffer solution (not containing Ca²⁺ or Mg²⁺) as the culture solution during separation is desirable from the perspective of facilitating separation.

### [Examples]

The present invention is described in greater detail through examples below.

### Example 1

### (1) Preparation of the electrode

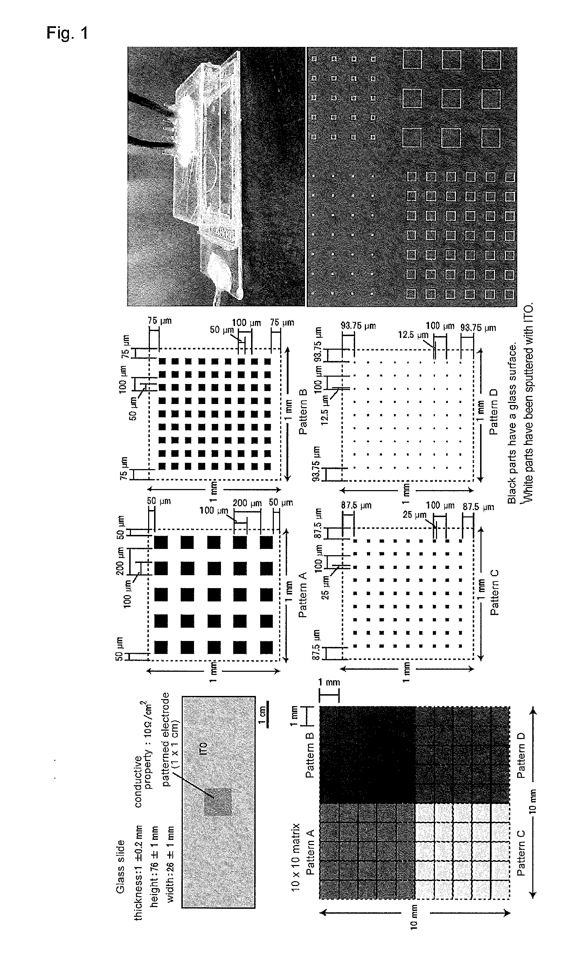

An electrode and a three-electrode culture system were prepared by the following procedure. Figure 1 shows drawings describing the procedure of preparing the electrode.

1) A patterned electrode (1 x 1 cm) coated with indium [tin] oxide (ITO) to 6.3 to 7.5 Ω/cm² in the center portion of a glass slide (upper left in Figure 1) was prepared (lower left in Figure 1). The patterned electrode, as shown on the lower left, was prepared with four separate regions. The patterns A to D of the individual regions are shown in the center of Figure 1. The black portions denote the glass surface, and the white portions denote the ITO electrode surface.
2) Using a diamond drill, a hole was opened in the edge portion of the glass slide and a connection was made between the wiring and the ITO electrode with an electrically conductive resin (Dotite, D-550, Fujikura Kasei (Ltd.)).
3) The chamber portion of a plastic chamber slide (Lab-tek chamber slide, 177410, NaigeNunc) was removed and silicon bond (Toshiba Silicone, TSE382-C clear), used for repairing aquariums, was employed to adhere it onto the ITO patterned electrode.
4) Holes were opened in two spots in the cover portion of the plastic chamber slide using a diamond drill, and connections were made with a platinum electrode and a silver/silver chloride electrode.
5) The cover and chamber were combined to complete a three-electrode culture system (photograph in upper right).

### (2) Adsorption of extracellular matrix protein

By the following procedure, extracellular matrix protein was caused to adsorb onto the surface of the electrode in the three-electrode culture system prepared in (1).

1) The ITO patterned electrode chamber was cleansed with ultrasound for five minutes in distilled water.
2) 1 M NaOH was charged to the ITO patterned electrode chamber and left standing for five minutes.
3) The ITO patterned electrode chamber was lightly washed with PBS(-) and then sterilized for five minutes on a clean bench under UV light.
4) Either 0.1 mg/mL of FITC-labeled poly-L-lysine (Sigma) in PBS(-) or 0.1 mg/mL of FITC-labeled collagen I (Sigma) in 0.01 M HCl aqueous solution was added to the chamber and the mixture was left standing at 37°C for 10 minutes.
5) The interior of the chamber was lightly rinsed twice with PBS(-), after which the ITO patterned electrode chamber was stored at 4°C or immediately employed for testing.

### (3) Application of potential

After causing the extracellular matrix protein to adsorb to the electrode surface in (2), the following procedure was used to apply a potential to cause the extracellular matrix protein to separate from the electrode surface.

1) A 5 mL quantity of PBS(-) was added to the ITO patterned electrode chamber.
2) The cover equipped with electrodes was put in place to prepare a three-electrode culture system.
3) A potentiostat (PS-14, Toho Technical Research) was used to apply a constant potential to cause separation of the extracellular matrix protein.
4) A light rinsing was conducted with PBS(-) following application of the constant potential. Subsequently, a confocal laser microscope (FV500, Olympus) was used to observe the distribution of the extracellular matrix protein.

### (4) Results

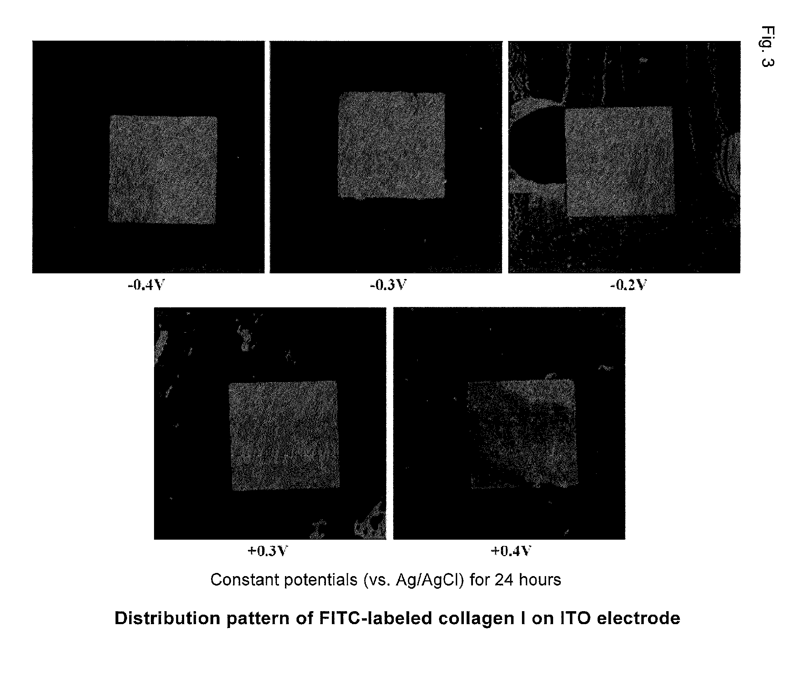

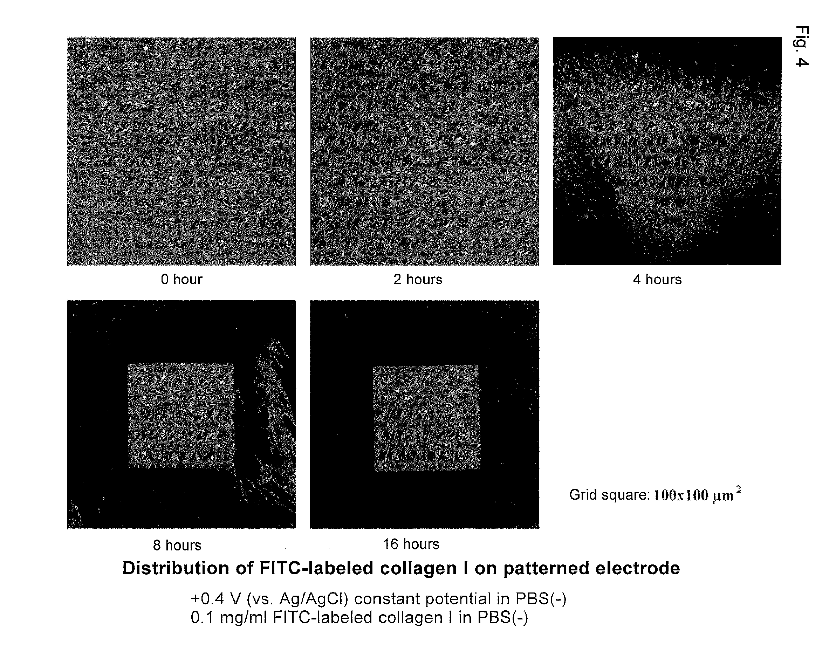

Figures 3 and 4 show the results when FITC-labeled collagen I was employed as the extracellular matrix protein. Figure 3 shows fluorescence images of FITC on the electrode surface obtained for an application for 24 hours of constant potentials (vs. Ag/AgCl) of -0.4 V, -0.3 V, -0.2 V, +0.3 V, and +0.4 V in PBS(-). The observation of fluorescence on the patterned glass surface (squares) indicated adsorption of the FITC-labeled collagen I. The fact that no fluorescence was observed on the surrounding ITO electrode portions indicated separation of the FITC-labeled collagen I.

Figure 4 shows fluorescence images of FITC on the surface of the electrode when the period of application was varied from 0 to 16 hours for a constant potential (vs. Ag/AgCl) of +0.4 V. At 0 to 8 hours, fluorescence was observed even in the ITO electrode portion around the patterned glass surface (squares). At 8 to 16 hours, fluorescence was only observed on the patterned glass surface (squares), indicating adsorption of the FITC-labeled collagen I. No fluorescence was observed in the surrounding ITO electrode portion, indicating separation of the FITC-labeled collagen I.

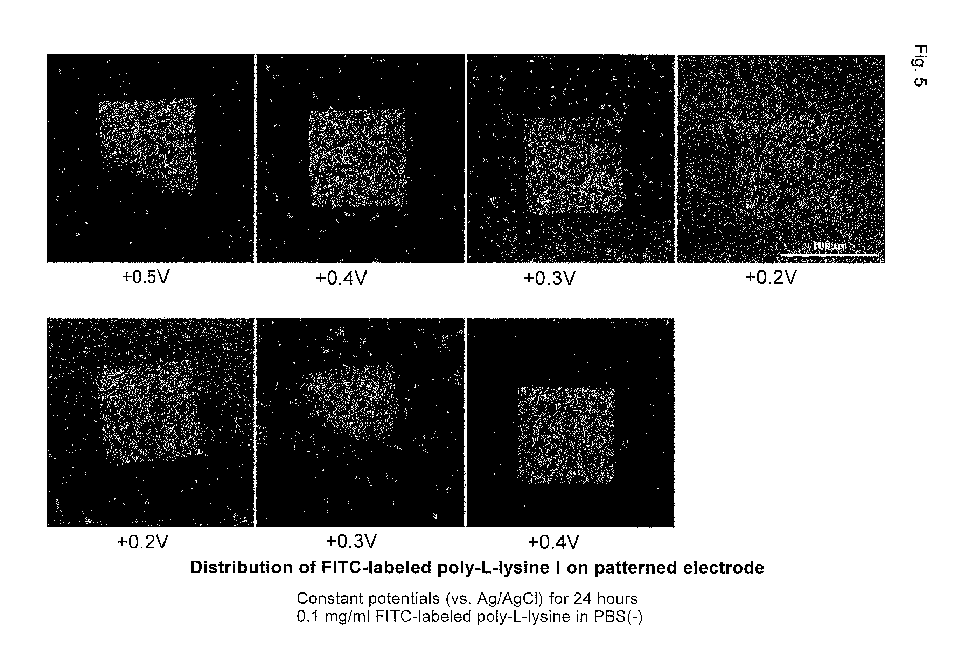

Figure 5 shows the results when FITC-labeled poly-L-lysine (Sigma) was employed as the extracellular matrix protein. Figure 5 shows fluorescence images of FITC on the electrode surface obtained for an application for 24 hours of constant potentials (vs. Ag/AgCl) of -0.4 V, -0.3 V, -0.2 V, +0.2 V, +0.3 V, +0.4 V, and +0.5 V in PBS(-). When constant potentials (vs. Ag/AgCl) of -0.4 V, -0.3 V, +0.3 V, +0.4 V, and +0.5 V were applied for 24 hours, fluorescence was observed on the patterned glass surface (square), indicating adsorption of the FITC-labeled poly-L-lysine. No fluorescence was observed in the surrounding ITO electrode portions, indicating separation of the FITC-labeled poly-L-lysine. When constant potentials (vs. Ag/AgCl) of -0.2 V and +0.2 V were applied for 24 hours, fluorescence was also observed in the ITO electrode portion surrounding the patterned glass surface (squares).

### Example 2

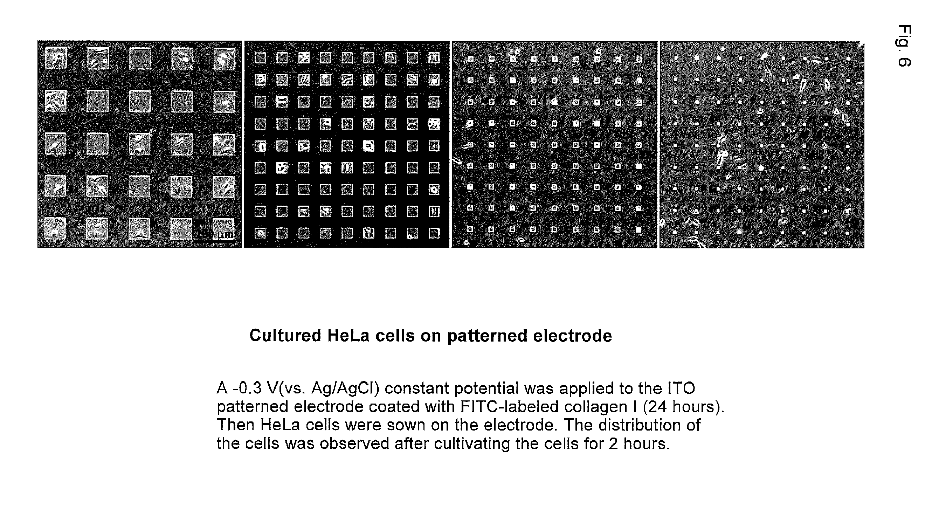

A 1 x 10⁵ cell/well quantity of animal cells (HeLa cells) suspended in serum-containing medium was inoculated into the chamber of the patterned electrode obtained by applying a constant potential of -0.3 V (vs. Ag/AgCl) in PBS(-) for 42 hours using FITC-labeled collagen I as the extracellular matrix protein in Example 1 and the cells were cultured for 24 hours. A -0.3 V constant potential was applied during the culture period (24 hours). Following application of the potential for 24 hours, the distribution of the cells and the extracellular matrix protein were observed by confocal laser microscopy (FV500, Olympus) and phase-contrast microscopy (CKX-41, Olympus). Figure 6 shows images of the electrode surface after 24 hours of culturing. It will be understood that animal cells (HeLa cells) adhered to the glass surface (square) to which the FITC-labeled collagen I had adsorbed. At 100 x 100 µm² and 50 x 50 µm², cells were successfully arranged on just the glass substrate. The fact that the arrangement of cells could be controlled down to 25 x 25 µm² was indicated.

### Example 3

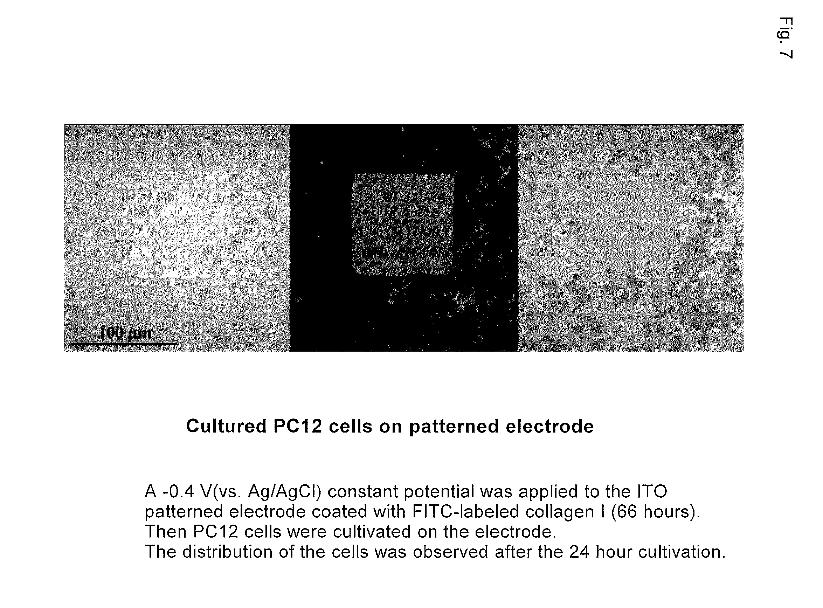

Animal cells (PC12 cells) suspended in serum-containing medium were inoculated into the chamber of a patterned electrode obtained by applying a constant potential of +0.4 V (vs. Ag/AgCl) in PBS(-) for 66 hours using FITC-labeled collagen I as the extracellular matrix protein in Example 1 and the cells were cultured for 24 hours. A +0.4 V constant potential was applied during the culture period (24 hours). Following application of the potential for 24 hours, the distribution of the cells and the extracellular matrix protein were observed by confocal laser microscopy (FV500, Olympus) and phase-contrast microscopy (CKX-41, Olympus). Figure 7 shows images of the electrode surface after 24 hours of culturing. It will be understood that animal cells (PC12 cells) had adhered to the ITO electrode substrate surface on which no FITC-labeled had adsorbed.

### Reference Example 1

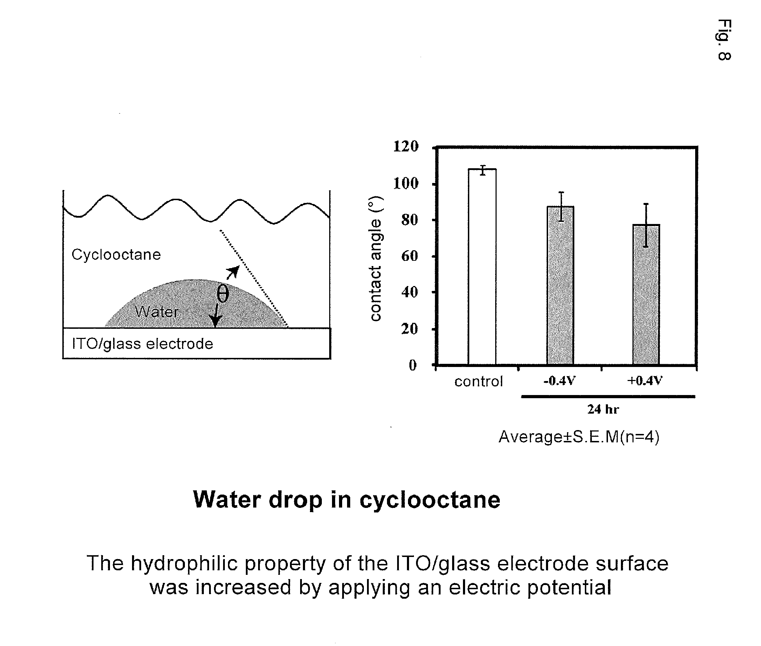

The water contact angle of the surface of the ITO electrode was measured when a constant voltage was being applied to the ITO electrode. The measurement was conducted by placing a water droplet on the surface of an ITO electrode in an electrolytic bath filled with cyclooctane and measuring the contact angle before and after the application of a constant potential (24 hours). The results are given in Figure 8. It will be understood that the application of constant potentials (vs. Ag/AgCl) of -0.4 V and +0.4 V caused the water contact angle to decrease by about 20 to 40%, increasing the hydrophilic property. Based on these results, it was presumed that the separation due to the application of a constant potential to the extracellular matrix protein in the present invention was partially due to the hydrophilic property of the electrode surface.

### [Industrial Applicability]

The present invention is useful in fields relating to the cultivation of animal cells.

## Claims

1. A method for preparing a substrate for arranging animal cells in an array, comprising the steps of:
(1) preparing a substrate having arrayed adsorption surfaces on an electrode substrate surface;
(2) causing an extracellular matrix to adsorb to the electrode surface and the adsorption surfaces of the substrate; and
(3) applying a weak potential to the electrode to cause the extracellular matrix on the electrode surface to separate to obtain a substrate with the extracellular matrix adhered to the adsorption surfaces thereof.

2. A method for preparing a substrate on which animal cells have been arranged in an array, comprising the steps of:
(1) preparing a substrate having adsorption surfaces in an array on an electrode substrate surface;
(2) causing an extracellular matrix to adsorb to the electrode surface and the adsorption surfaces of the substrate;
(3) applying a weak potential to the electrode to cause the extracellular matrix on the electrode surface to separate to obtain a substrate with the extracellular matrix adhered to the adsorption surfaces thereof;
(4) culturing the animal cells on the surface of the substrate obtained in (3) to obtain a substrate on which the animal cells have adhered to the adsorption surfaces thereof.

3. The preparation method according to claim 1 or 2, wherein the dimensions of each of the adsorption surfaces in the array permit the adhesion of a single animal cell to each adsorption surface.

4. The preparation method according to any one of claims 1 to 3 wherein the substrate is selected from the group consisting of electrically non-conductive organic materials and inorganic materials.

5. The preparation method according to any one of claims 1 to 3 wherein the substrate is glass, plastic, or ceramic.

6. The preparation method according to any one of claims 1 to 4, wherein the weak potential in (3) falls within a range of -0.4 V to -0.2 V or +0.2 V to + 0.5 V (vs. Ag/Cl).

7. The preparation method according to any one of claims 2 to 6, wherein the animal cell culturing in (4) is conducted while applying a potential falling within a range of -0.4 V to -0.2 V (vs. Ag/AgCl) to the electrode to inhibit adhesion of animal cells to the electrode.

8. The preparation method according to any one of claims 2 to 6, wherein the animal cell culturing in (4) is conducted by applying a positive potential to the electrode to cause animal cells to adsorb to the electrode, yielding a substrate on which the animal cells have adhered to the adsorption surfaces and the electrode.
